(19) Europäisches Patentamt
European Patent Office
Office européen des brevets

(11) Veröffentlichungsnummer : **0 146 837**
**B1**

# (12) EUROPÄISCHE PATENTSCHRIFT

(45) Veröffentlichungstag der Patentschrift : **23.03.88**

(51) Int. Cl.[4] : **C 07 D333/22**, C 07 D333/24, C 07 D333/48, A 01 N 43/10

(21) Anmeldenummer : **84114755.6**

(22) Anmeldetag : **04.12.84**

(54) **Cyclohexenonderivate, Verfahren zu ihrer Herstellung und ihre Verwendung zur Bekämpfung unerwünschten Pflanzenwuchses.**

(30) Priorität : **08.12.83 DE 3344352**
**10.08.84 DE 3429437**

(43) Veröffentlichungstag der Anmeldung : **03.07.85 Patentblatt 85/27**

(45) Bekanntmachung des Hinweises auf die Patenterteilung : **23.03.88 Patentblatt 88/12**

(84) Benannte Vertragsstaaten : **BE CH DE FR GB IT LI NL**

(56) Entgegenhaltungen :
**EP-A- 0 070 370**
**EP-A- 0 071 707**
**EP-A- 0 115 808**

(73) Patentinhaber : **BASF Aktiengesellschaft**
**Carl-Bosch-Strasse 38**
**D-6700 Ludwigshafen (DE)**

(72) Erfinder : **Keil, Michael, Dr.**
**Fontanestrasse 4**
**D-6713 Freinsheim (DE)**
Erfinder : **Jahn, Dieter, Dr.**
**Burgunder Weg 8**
**D-6803 Edingen-Neckarhausen (DE)**
Erfinder : **Becker, Rainer, Dr.**
**Im Haseneck 22**
**D-6702 Bad Duerkheim (DE)**
Erfinder : **Goetz, Norbert, Dr.**
**Schoefferstrasse 25**
**D-6520 Worms 1 (DE)**
Erfinder : **Wuerzer, Bruno, Dr.**
**Ruedigerstrasse 13**
**D-6701 Otterstadt (DE)**
Erfinder : **Meyer, Norbert, Dr.**
**Dossenheimer Weg 22**
**D-6802 Ladenburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 146 837 B1

Jouve, 18, rue St-Denis, 75001 Paris, France

**Beschreibung**

Die Erfindung betrifft neue Cyclohexenonderivate, Verfahren zu ihrer Herstellung, sowie Herbizide, die diese Verbindungen als Wirkstoffe enthalten.

Aus EP-A-00 71 707 sind eine Reihe von Cyclohexenonen, die in 5-Stellung durch bestimmte nichtaromatische Heterocyclen substituiert sind, als Herbizide bekannt. Aus der EP-A-115 808 sind insbesondere speziell in 5-Stellung direkt heterocyclisch substituierte Cyclohexenone bekannt, wobei der Heterocyclus Sulfinyl- oder Sulfonylgruppen als Ringelemente aufweist und aus der EP-A-70 370 entsprechend mit cyclischen Ethern bzw. Acetalen substituierte Cyclohexenone.

Es wurde gefunden, daß Cyclohexenonderivate der Formel

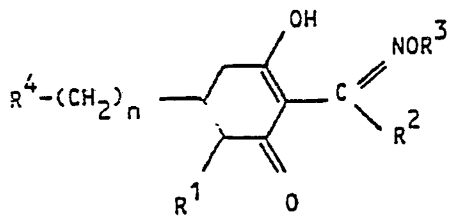

(I)

in der

$R^1$ Wasserstoff oder Alkoxycarbonyl mit 2 bis 5 Kohlenstoffatomen,

$R^2$ Alkyl mit 1 bis 4 Kohlenstoffatomen,

$R^3$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen,

$R^4$ einen nichtaromatischen, gegebenenfalls eine Doppelbindung enthaltenden 5-Ring, der ein Schwefelatom, eine Sulfinyl- oder eine Sulfonylgruppe als Ringglied enthält und gegebenenfalls durch 1 bis 3 Alkylgruppen mit 1 bis 4 Kohlenstoffatomen substituiert ist und

n 0, 1 oder 2 bedeuten,

mit der Maßgabe, daß n nicht 0 ist, wenn $R^4$ einen 5-Ring mit einer Sulfinyl- oder einer Sulfonylgruppe bedeutet, deren Gleichgewichtsisomere (Tautomere) und Salze dieser Verbindungen herbizid wirksam sind.

Die Cyclohexenonderivate der Formel I können in mehreren Formen auftreten, die alle vom Patentanspruch umfaßt werden :

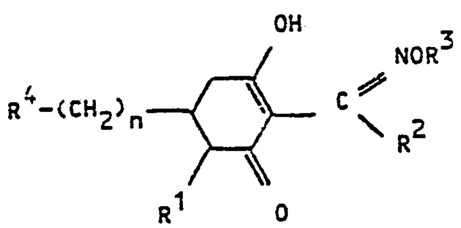

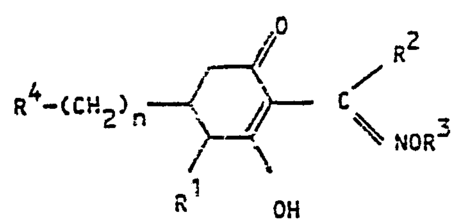

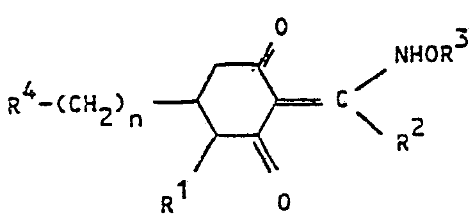

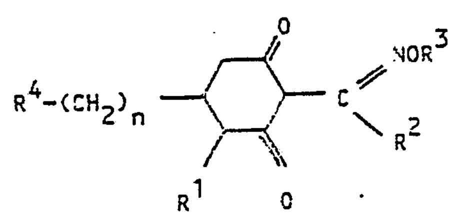

$R^1$ in Formel I bedeutet Wasserstoff oder Alkoxycarbonyl mit 1 bis 4 Kohlenstoffatomen, insbesondere Methoxycarbonyl. $R^2$ in Formel I steht für unverzweigte oder verzweigte Alkylreste mit 1 bis 4 Kohlenstoffatomen, d. h. für Methyl, Ethyl, n-Propyl, i-Propyl, n-Butyl, sec.-Butyl, i-Butyl, tert.-Butyl.

Reste für $R^3$ in Formel I sind Propargyl, Alkyl mit 1 bis 4 C-Atomen, Alkenyl mit 3 oder 4 Kohlenstoffatomen oder Halogenalkenyl mit 3 oder 4 Kohlenstoffatomen, das bis zu drei Halogensubstituenten enthalten kann, beispielsweise Methyl, Ethyl, n-Propyl, i-Propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorpro-1-en-3-yl.

$R^4$ in Formel I bedeutet einen nichtaromatischen, gegebenenfalls eine Doppelbindung enthaltenden 5-Ring, der ein Schwefelatom, eine Sulfinyl- oder eine Sulfonylgruppe als Ringglied enthält und

gegebenenfalls durch 1 bis 3 Alkylgruppen mit 1 bis 4 Kohlenstoffatomen, vorzugsweise Methyl, Ethyl oder n-Propyl, substituiert ist. Beispiele für $R^4$ sind Tetrahydrothienylreste, 2,5-Dihydrothienylreste, 1-Oxo-tetrahydrothienylreste, 1-Oxo-2,5-dihydrothienylreste, 1,1-Dioxo-2,5-Dihydrothienylreste oder 1,1-Dioxo-tetrahydrothienylreste, z. B. Tetrahydrothien-3-yl, 2,5--Dihydrothien-3-yl, 1-Oxo-tetrahydrothien-3-yl, 2,5-Dihydro-1-oxo-thien-3-yl, 1,1-Dioxo-tetrahydrothien-3-yl, 2,5-Dihydro-1,1-dioxo-thien-3-yl, 2-Methyl-tetrahydrothien-3-yl, 2,5-Dihydro-2-methyl-1-oxo-thien-3-yl, 2,2-Dimethyltetrahydrothien-3-yl, 2,5-Dihydro-2,2-dimethylthien-3-yl, 2,2-Dimethyl-1-oxo-tetrahydrothien-3-yl, 2,5-Dihydro-2,2-dimethyl-1-oxo-thien-3-yl, 2,2-Dimethyl-1,1-dioxo-tetrahydrothien-3-yl, 2,5-Dihydro-2,2-dimethyl-1,1-dioxo-thien-3-yl, Tetrahydrothien-2-yl, 1-Oxotetrahydrothien-2-yl, 1,1-Dioxo-tetrahydrothien-2-yl, 4,5-Dihydro-4-methylthien-2-yl, 4,5-Dihydro-4-methyl-1-oxo-thien-2-yl-, 4,5-Dihydro-1,1-dioxo-4-methylthien-2-yl, 4-Methyl-tetrahydrothien-2-yl, 4-Methyl-1-oxo-tetrahydrothien-2-yl, 4-Methyl-1,1-dioxo-tetrahydrothien-2-yl,4,5-Dihydro-4,5-dimethyl-thien-2-yl, 4,5-Dihydro-4,5-dimethyl-1-oxo-thien-2-yl, 4,5-Dihydro-4,5-dimethyl-1,1-dioxo-thien-2-yl, 4,5-Dimethyl-tetrahydrothien-2-yl, 4,5-Dimethyl-1-oxo-tetrahydrothien-2-yl, 4,5-Dimethyl-1,1-dioxotetrahydrothien-2-yl, 4,5-Dihydro-4-ethyl-5-n-propyl-thien-2-yl, 4,5-Dihydro-4-ethyl-1-oxo-5-n-propyl-thien-2-yl, 4,5-Dihydro-1,1-dioxo-4-ethyl-5-n-propyl-thien-2-yl, 4-Ethyl-5-n-propyl-tetrahydrothien-2-yl, 4-Ethyl-1-oxo-5-n-propyl-tetrahydrothien-2-yl, 1,1-Dioxo-4-ethyl-5-n-propyltetrahydrothien-2-yl.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Batriumsalze, Mangan-, Kupfer, Zink- oder Eisensalze sowie Ammonium-, Phosphonium-, Sulfonium- und Sulfoxoniumsalze, beispielsweise Ammoniumsalze, Tetraalkylammoniumsalze, Benzyltrialkylammoniumsalze, Trialkylsulfoniumsalze, Trialkylsulfoxoniumsalze in Betracht.

Die Cyclohexenonderivate der Formel I können durch Umsetzung von Verbindungen der Formel

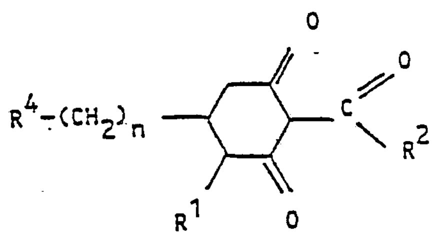

(II)

in der $R^1$, $R^2$, $R^4$ und n die obengenannten Bedeutungen haben, mit Ammoniumverbindungen der Formel $R^3O—NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9. Die Einstellung des pH-Bereiches erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^3O—NH_3Y$, zugesetzt.

Als Lösungsmittel sind beispielsweise Dimethylsulfoxid, Alkohole, wie Methanol, Ethanol, Isopropanol, Benzol, Toluol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^3O—NH_2$, in der $R^3$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin in Form einer wäßrigen Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, Toluol, erhalten werden. Anstelle der Hydroxide können auch Natrium- und Kaliumalkoholate zur Herstellung der Alkalimetallsalze dienen.

Die übrigen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium- und Phosphoniumsalze können durch Umsetzung von Verbindungen der Formel I mit Ammonium-, Phosphonium-, Sulfonium- oder Sulfoxoniumhydroxiden gegebenenfalls in wäßriger Lösung erhalten werden.

Die Verbindungen der Formel II können aus Cyclohexan-1,3-dionen der Formel III, die auch in den tautomeren Formeln IIIa und IIIb vorliegen können,

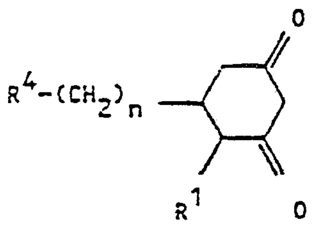

(III)

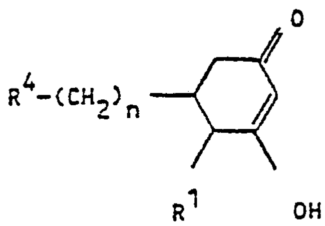

(IIIa)

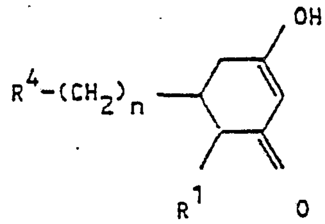

(IIIb)

nach literaturbekannten Methoden (Tetrahydron Letters, 29, 2491 (1975)) hergestellt werden.

Es ist auch möglich, Verbindungen der Formel II über die Zwischenstufe der Enolester, die bei der Umsetzung von Verbindungen der Formel II eventuell als Isomerengemische anfallen und in Gegenwart von Imidazol- oder Pyridinderivaten umgelagert werden (JP-OS 79/063052), herzustellen.

Zu den Verbindungen der Formel III gelangt man nach literaturbekannten Verfahren, wie dies aus folgendem Schema hervorgeht :

(Siehe Schema Seite 5 f.)

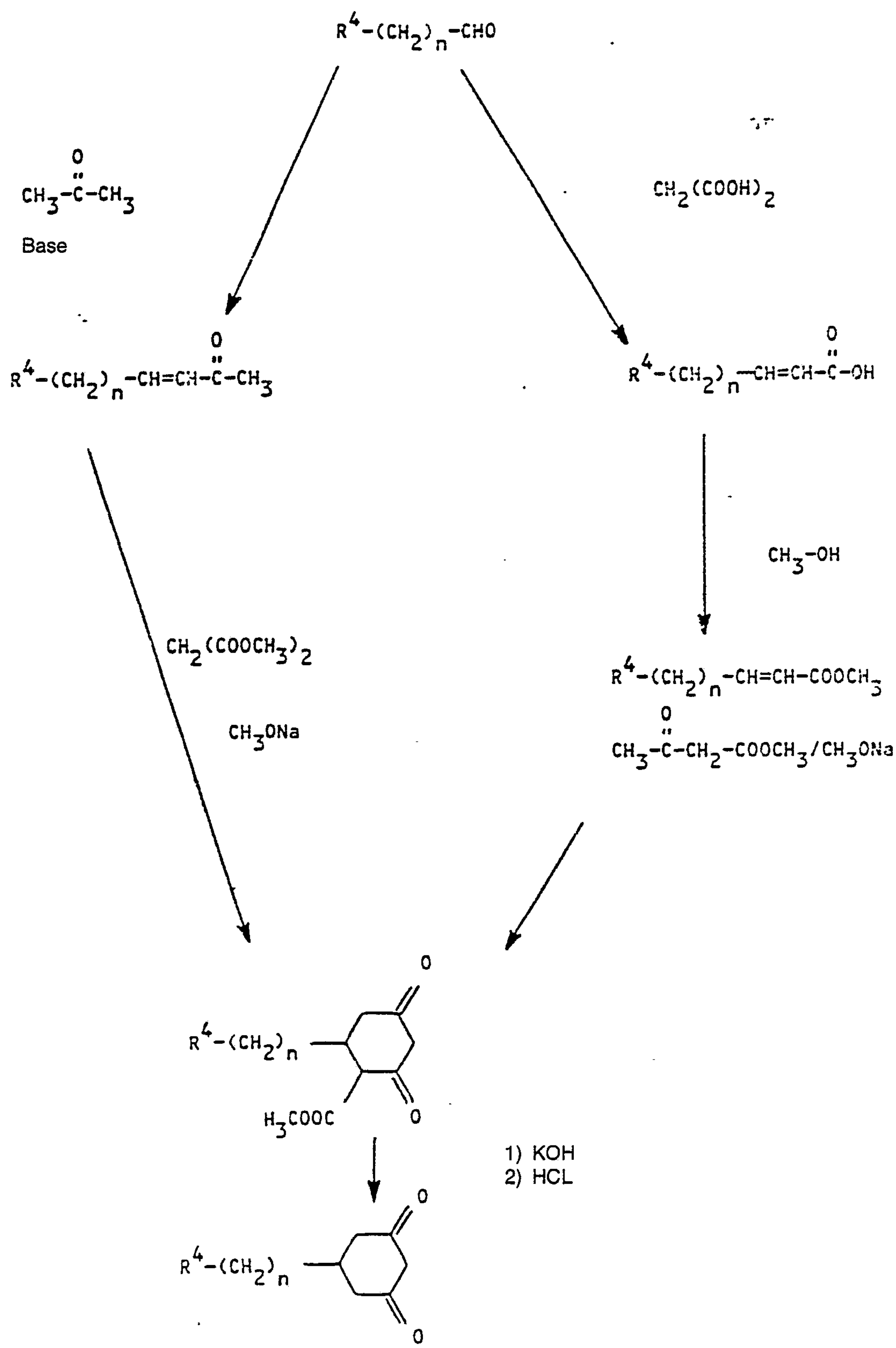
Base
$CH_2(COOH)_2$
$CH_3-OH$
$CH_2(COOCH_3)_2$
$CH_3ONa$
$CH_3-CO-CH_2-COOCH_3/CH_3ONa$
1) KOH
2) HCL

Die als Ausgangsverbindungen benötigten Aldehyde $R^4-(CH_2)_n-CHO$ sind nicht alle literaturbekannt, sie werden durch Anwendung allgemein bekannter Methoden, zum Beispiel durch Reduktion von Carbonsäurederivaten und Nitrilen, Grignard-Reaktionen, Verseifung und Decarboxylierung von Glycidestern, Umsetzung mit Wittig-Reagentien und Methyl-methylthiomethylsulfoxid, Spaltung geeigneter Epoxide oder Acetale erhalten. Auch Substitutionen, Kondensationen oder Reduktionen geeigneter Vorstufen führten zum Erfolg, wie an den nachfolgenden Beispielen demonstriert werden soll :

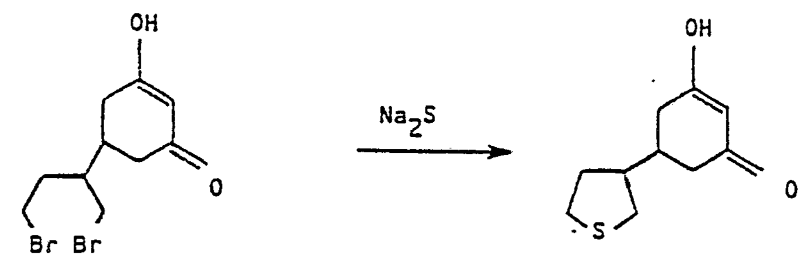


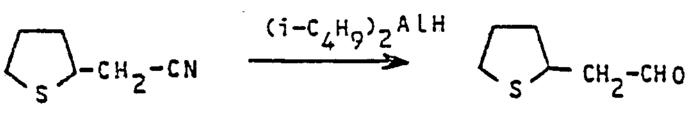


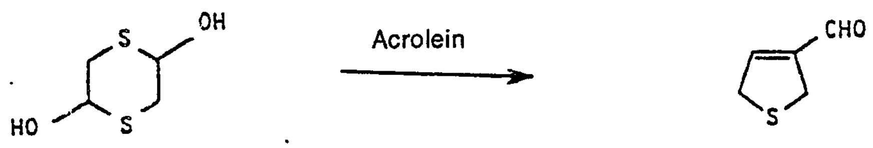


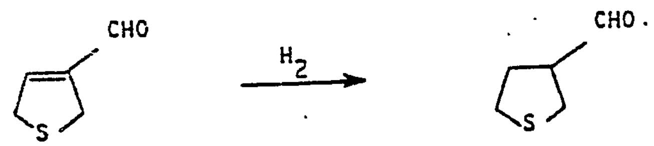


Man erhält z. B. den Tetrahydrothien-3-yl-acetaldehyd auf folgende Weise :

8,0 g Tetrahydrothien-2-yl-acetonitril (Bull. Soc. Chim. France 1974, 590) werden in 80 ml absolutem Toluol vorgelegt und bei —70 °C unter Stickstoffatmosphäre tropfenweise mit 58 ml einer 20 %igen Lösung von Diisobutylammoniumhydrid in Toluol versetzt. Nach 30 minütigem Rühren bei —60 °C läßt man die Reaktionsmischung auf Raumtemperatur kommen und gibt unter äußerer Kühlung vorsichtig tropfenweise Methanol zu (ca. 8 ml). Nach einstündigem Rühren wird die Mischung in 400 ml gesättigte Ammoniumchlorid-Lösung eingerührt und mit 250 ml 5 %iger Schwefelsäure versetzt. Die organische Phase wird abgetrennt, zweimal mit Wasser extrahiert und über Natriumsulfat getrocknet. Nach Einengen und Destillation des Rückstandes bei 40 bis 42 °C/0,4 mbar werden 3,7 g Tetrahydrothien-3-yl-acetaldehyd als wasserklare Flüssigkeit erhalten.

Die Verbindungen der Formel I können auch durch Oxidation von entsprechenden Vorstufen, in denen der Schwefel eine niedrigere Oxidationsstufe besitzt, erhalten werden. Oxidationsmittel sind beispielsweise Sauerstoff, Ozon, Peroxyverbindungen, wie Wasserstoffperoxide, Persäuren, Hydroperoxide, Halogene, anorganische Halogenverbindungen, wie Hypochlorit, Chlorat, Stickstoffverbindungen, wie Salpetersäure und Distickstoffpentoxid, Salze von Metallen höherer Wertigkeit, wie Blei-, Wismut-, Vanadin-, Mangan-, Chrom-, Kobaltsalze. Auch die anodische Oxidation ist möglich. Die Oxidation kann nicht nur auf der Stufe der Oximether durchgeführt werden, sondern ist prinzipiell auf jeder Stufe des vorstehend beschriebenen Syntheseweges möglich.

Die Identifizierung und Charakterisierung der Verbindungen der Formel I erfolgen am geeignetsten mit Hilfe der Protonenkernresonanzspektroskopie. In den nachstehenden Beispielen sind daher einige strukturspezifische $^1H$-NMR-Daten angegeben (Lösungsmittel $CDCl_3$, interner Standard Tetramethylsilan ; s = Singulett, d = Dublett, t = Triplett, q = Quartett, m = Multiplett).

Die folgenden Beispiele erläutern die Herstellung der Cyclohexenonderivate der Formel I :

Beispiel 1

4,2 g 2-Butyryl-5-(2,5-dihydro-2,2-dimethyl-thien-3-yl)-3-hydroxy-cyclohex-2-en-1-on, 1,6 g Ethoxyammoniumchlorid, 1,3-g Natriumhydrogencarbonat und 50 ml Methanol werden bei Raumtemperatur 16 h gerührt. Das Lösungs mittel wird unter vermindertem Druck abdestilliert, zum verbleibenden Rückstand werden jeweils 50 ml Wasser und Dichlormethan gegeben, nach dem Rühren die Phasen getrennt und die organische Phase über Natriumsulfat getrocknet. Nach Abdestillieren des Lösungsmittels unter vermindertem Druck erhält man 3,5 g (5-(2,5-Dihydro-2,2-dimethyl-thien-3-yl)-2-(1-ethoxyiminobutyl)-3-hydroxy-cyclohex-2-en-1-on als gelbliches Öl.

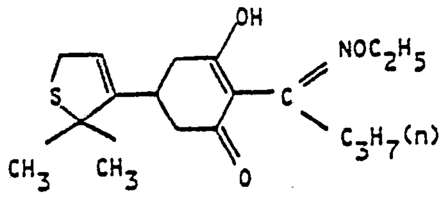

(ppm) : 1,51 (s, 6H) ; 3,68 (d, 2H) ; 4,15 (q, 2H), 5,63 (t, 1H).

Beispiel 2

3,0 g 2-(1-Ethoxyiminobutyl)-3-hydroxy-5-(tetrahydrothien-2-ylmethyl)-cyclohex-2-en-1-on werden in 200 ml Chloroform gelöst. Zu dieser Lösung wird bei 5 °C eine Lösung von 3,8 g 85 %iger m-Chlorperbenzoesäure zugetropft. Man läßt die Reaktionsmischung auf Raumtemperatur kommen, saugt von gebildeten Niederschlag ab und extrahiert mit halbgesättigter Natriumhydrogencarbonat-Lösung. Nach Trocknen über Natriumsulfat wird das Lösungsmittel unter vermindertem Druck abdestilliert. Der verbleibende Rückstand wird in Toluol aufgenommen, mit 10 %iger Natronlauge extrahiert, die wäßrige Phase wird abgetrennt und mit verd. Salzsäure auf pH 4 gestellt. Nach Extraktion mit Methylenchlorid und Einengen der organischen Phase erhält man 5-(1,1-Dioxo-tetrahydrothien-2-ylmethyl)-2-(1-ethoxyimino-butyl)-3-hydroxy-cyclohex-2-en-1-on.

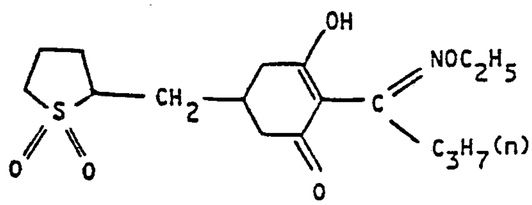

(Siehe Tabellen Seite 8 ff.)

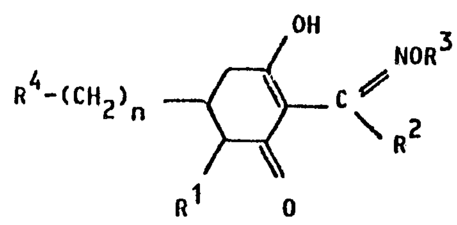

(I)

| Nr. | $R^4$ | n | $R^1$ | $R^2$ | $R^3$ | $^1$H-NMR (ppm) |
|---|---|---|---|---|---|---|
| 1 | Tetrahydrothien-3-yl | 0 | H | $n-C_3H_7$ | $C_2H_5$ | 0,97(t,3H); 1,33(t,3H); 2,91(m,4H); 4,12(q,2H) |
| 2 | Tetrahydrothien-3-yl | 0 | H | $n-C_3H_7$ | $CH_2-CH=CH_2$ | 0,97(t,3H); 2,92(m,4H); 4,55(d,2H); 5,34(m,2H); 5,97(m,1H) |
| 3 | Tetrahydrothien-3-yl | 0 | H | $C_2H_5$ | $CH_2-CH=CH_2$ | 1,13(t,3H); 2,92(m,4H); 4,54(d,2H); 5,35(m,2H); 5,97(m,1H) |
| 4 | Tetrahydrothien-3-yl | 0 | H | $C_2H_5$ | $C_2H_5$ | 1,14(t,3H); 1,33(t,3H); 2,95(m,4H); 4,11(q,2H) |
| 5 | Tetrahydrothien-3-yl | 0 | H | $CH_3$ | $C_2H_5$ | |
| 6 | Tetrahydrothien-3-yl | 0 | H | $CH_3$ | $n-C_3H_7$ | |
| 7 | Tetrahydrothien-3-yl | 0 | H | $n-C_3H_7$ | $n-C_3H_7$ | 0,97(t,3H); 2,98(m,4H); 4,00(t,2H) |
| 8 | Tetrahydrothien-3-yl | 0 | H | $n-C_3H_7$ | $CH_2-C=CH$ | 0,97(t,3H); 2,51(s,1H); 4,34(s,1H) |
| 9 | Tetrahydrothien-3-yl | 0 | H | $n-C_3H_7$ | $CH_2-CH=CHCl$ (trans) | 0,97(t,3H); 2,95(m,4H); 4,52(d,2H); 6,10(m,1H); 6,35(d,1H) |
| 10 | Tetrahydrothien-3-yl | 0 | H | $n-C_3H_7$ | $CH_2-CCl=CCl_2$ | 0,97(t,3H); 2,95(m,4H); 4,94(s,2H) |
| 11 | Tetrahydrothien-3-yl | 0 | $COOCH_3$ | $n-C_3H_7$ | $C_2H_5$ | |
| 12 | Tetrahydrothien-3-yl | 0 | $COOCH_3$ | $n-C_3H_7$ | $CH_2-CH=CH_2$ | |
| 13 | Tetrahydrothien-3-yl | 0 | $COOCH_3$ | $C_2H_5$ | $CH_2-CH=CH_2$ | |

(Fortsetzung)

| Nr. | $R^4$ | n | $R^1$ | $R^2$ | $R^3$ | $^1$H-NMR (ppm) |
|---|---|---|---|---|---|---|
| 14 | Tetrahydrothien-3-yl | 0 | $COOCH_3$ | $C_2H_5$ | $C_2H_5$ | |
| 15 | Tetrahydrothien-3-yl | 1 | H | $n-C_3H_7$ | $C_2H_5$ | |
| 16 | Tetrahydrothien-3-yl | 1 | H | $n-C_3H_7$ | $CH_2-CH=CH_2$ | |
| 17 | Tetrahydrothien-3-yl | 1 | H | $C_2H_5$ | $CH_2-CH=CH_2$ | |
| 18 | Tetrahydrothien-3-yl | 1 | H | $C_2H_5$ | $C_2H_5$ | |
| 19 | 2,5-Dihydrothien-3-yl | 0 | H | $n-C_3H_7$ | $C_2H_5$ | 0,97(t,3H); 1,32(t,3H); 4,10(q,2H); 5,60(1H) |
| 20 | 2,5-Dihydrothien-3-yl | 0 | H | $n-C_3H_7$ | $CH_2-CH=CH_2$ | |
| 21 | 2,5-Dihydrothien-3-yl | 0 | H | $C_2H_5$ | $CH_2-CH=CH_2$ | |
| 22 | 2,5-Dihydrothien-3-yl | 0 | H | $C_2H_5$ | $C_2H_5$ | |
| 23 | 1-Oxotetrahydrothien-3-yl | 1 | H | $n-C_3H_7$ | $C_2H_5$ | |
| 24 | 1-Oxotetrahydrothien-3-yl | 1 | H | $n-C_3H_7$ | $CH_2-CH=CH_2$ | |
| 25 | 1-Oxotetrahydrothien-3-yl | 1 | H | $C_2H_5$ | $CH_2-CH=CH_2$ | |
| 26 | 1-Oxotetrahydrothien-3-yl | 1 | H | $C_2H_5$ | $C_2H_5$ | |
| 27 | 1,1-Dioxotetrahydrothien-3-yl | 1 | H | $n-C_3H_7$ | $C_2H_5$ | |
| 28 | 1,1-Dioxotetrahydrothien-3-yl | 1 | H | $n-C_3H_7$ | $CH_2CH=CH_2$ | |
| 29 | 1,1-Dioxotetrahydrothien-3-yl | 1 | H | $C_2H_5$ | $CH_2CH=CH_2$ | |
| 30 | 1,1-Dioxotetrahydrothien-3-yl | 1 | H | $C_2H_5$ | $C_2H_5$ | |
| 31 | 2-Methyl-tetrahydrothien-3-yl | 0 | H | $n-C_3H_7$ | $C_2H_5$ | 0,97(t,3H); 3,25(m,1H); 4,10(q,2H) |
| 32 | 2-Methyl-tetrahydrothien-3-yl | 0 | H | $n-C_3H_7$ | $CH_2-CH=CH_2$ | 0,97(t,3H); 1,34(d,3H); 3,24(m,1H); 4,53(d,2H) |
| 33 | 2-Methyl-tetrahydrothien-3-yl | 0 | H | $C_2H_5$ | $CH_2-CH=CH_2$ | |
| 34 | 2-Methyl-tetrahydrothien-3-yl | 0 | H | $C_2H_5$ | $C_2H_5$ | |
| 35 | 2,2-Dimethyltetrahydrothien-3-yl | 0 | $COOCH_3$ | $n-C_3H_7$ | $C_2H_5$ | |
| 36 | 2,2-Dimethyltetrahydrothien-3-yl | 0 | $COOCH_3$ | $n-C_3H_7$ | $CH_2-CH=CH_2$ | |

(Fortsetzung)

| Nr. | $R^4$ | n | $R^1$ | $R^2$ | $R^3$ | $^1H$-NMR (ppm) |
|---|---|---|---|---|---|---|
| 37 | 2,2-Dimethyltetrahydrothien-3-yl | 0 | H | $n-C_3H_7$ | $C_2H_5$ | 0,98(t,3H); 1,36(s,3H); 1,52(s,3H); 4,10(q,2H) |
| 38 | 2,2-Dimethyltetrahydrothien-3-yl | 0 | H | $n-C_3H_7$ | $CH_2-CH=CH_2$ | 0,97(t,3H); 1,36(s,3H); 1,52(s,3H); 4,53(d,2H) |
| 39 | 2,2-Dimethyltetrahydrothien-3-yl | 0 | H | $C_2H_5$ | $CH_2-CH=CH_2$ | 1,16(t,3H); 1,35(s,3H); 1,52(s,3H); 4,54(d,2H) |
| 40 | 2,2-Dimethyltetrahydrothien-3-yl | 0 | | $C_2H_5$ | $C_2H_5$ | 1,16(t,3H); 1,36(s,3H); 1,52(s,3H); 4,13(q,2H) |
| 41 | 2,2-Dimethyl-2,5-dihydrothien-3-yl | 0 | H | $n-C_3H_7$ | $C_2H_5$ | 1,51(s,6H); 3,68(d,2H); 4,15(q,2H); 5,63(t,1H) |
| 42 | 2,2-Dimethyl-2,5-dihydrothien-3-yl | 0 | H | $n-C_3H_7$ | $CH_2-CH=CH_2$ | 1,51(s,6H); 3,65(d,2H); 4,55(d,2H); 5,95(m,1H) |
| 43 | 2,2-Dimethyl-2,5-dihydrothien-3-yl | 0 | H | $C_2H_5$ | $CH_2-CH=CH_2$ | |
| 44 | 2,2-Dimethyl-2,5-dihydrothien-3-yl | 0 | H | $C_2H_5$ | $C_2H_5$ | |
| 45 | 2,2-Dimethyl-2,5-dihydrothien-3-yl | 0 | $COOCH_3$ | $n-C_3H_7$ | $C_2H_5$ | |
| 46 | 2,2-Dimethyl-2,5-dihydrothien-3-yl | 0 | $COOCH_3$ | $n-C_3H_7$ | $CH_2-CH=CH_2$ | |
| 47 | 2,2-Dimethyl-2,5-dihydrothien-3-yl | 0 | $COOCH_3$ | $C_2H_5$ | $CH_2-CH=CH_2$ | |
| 48 | 2,2-Dimethyl-2,5-dihydrothien-3-yl | 0 | $COOCH_3$ | $C_2H_5$ | $C_2H_5$ | |
| 49 | Tetrahydrothien-2-yl | 0 | H | $n-C_3H_7$ | $C_2H_5$ | |
| 50 | Tetrahydrothien-2-yl | 0 | H | $n-C_3H_7$ | $CH_2-CH=CH_2$ | |
| 51 | Tetrahydrothien-2-yl | 0 | H | $C_2H_5$ | $CH_2-CH=CH_2$ | |
| 52 | Tetrahydrothien-2-yl | 0 | H | $C_2H_5$ | $C_2H_5$ | |
| 53 | Tetrahydrothien-2-yl | 1 | H | $n-C_3H_7$ | $C_2H_5$ | 1,35(t, ); 2,85(m,4H); 3,45(m,1H); 4,10(q,2H) |
| 54 | Tetrahydrothien-2-yl | 1 | H | $n-C_3H_7$ | $CH_2-CH=CH_2$ | |
| 55 | Tetrahydrothien-2-yl | 1 | H | $C_2H_5$ | $CH_2-CH=CH_2$ | |
| 56 | Tetrahydrothien-2-yl | 1 | H | $C_2H_5$ | $C_2H_5$ | |

(Fortsetzung)

| Nr. | $R^4$ | n | $R^1$ | $R^2$ | $R^3$ | $^1H$-NMR (ppm) |
|---|---|---|---|---|---|---|
| 57 | Tetrahydrothien-2-yl | 2 | H | $n-C_3H_7$ | $C_2H_5$ | |
| 58 | Tetrahydrothien-2-yl | 2 | H | $n-C_3H_7$ | $CH_2-CH=CH_2$ | |
| 59 | Tetrahydrothien-2-yl | 2 | H | $C_2H_5$ | $CH_2-CH=CH_2$ | |
| 60 | Tetrahydrothien-2-yl | 2 | H | $C_2H_5$ | $C_2H_5$ | |
| 61 | 1-Oxotetrahydrothien-2-yl | 1 | H | $n-C_3H_7$ | $C_2H_5$ | 0,95(t,3H); 1,35(t,3H); 1,45-3,50(18H); 4,05(q,2H) |
| 62 | 1-Oxotetrahydrothien-2-yl | 1 | H | $n-C_3H_7$ | $CH_2-CH=CH_2$ | |
| 63 | 1-Oxotetrahydrothien-2-yl | 1 | H | $C_2H_5$ | $CH_2-CH=CH_2$ | |
| 64 | 1-Oxotetrahydrothien-2-yl | 1 | H | $C_2H_5$ | $C_2H_5$ | |
| 65 | 1-Oxotetrahydrothien-2-yl | 2 | H | $n-C_3H_7$ | $C_2H_5$ | |
| 66 | 1-Oxotetrahydrothien-2-yl | 2 | H | $n-C_3H_7$ | $CH_2-CH=CH_2$ | |
| 67 | 1-Oxotetrahydrothien-2-yl | 2 | H | $C_2H_5$ | $CH_2-CH=CH_2$ | |
| 68 | 1-Oxotetrahydrothien-2-yl | 2 | H | $C_2H_5$ | $C_2H_5$ | |
| 69 | 1-Oxotetrahydrothien-2-yl | 1 | H | $n-C_3H_7$ | $C_2H_5$ | |
| 70 | 1-Oxotetrahydrothien-2-yl | 1 | H | $n-C_3H_7$ | $CH_2-CH=CH_2$ | |
| 71 | 1-Oxotetrahydrothien-2-yl | 1 | H | $C_2H_5$ | $CH_2-CH=CH_2$ | |
| 72 | 1-Oxotetrahydrothien-2-yl | 1 | H | $C_2H_5$ | $C_2H_5$ | |
| 73 | 1,1-Dioxotetrahydrothien-2-yl | 2 | H | $n-C_3H_7$ | $C_2H_5$ | |
| 74 | 1,1-Dioxotetrahydrothien-2-yl | 2 | H | $n-C_3H_7$ | $CH_2-CH=CH_2$ | |
| 75 | 1,1-Dioxotetrahydrothien-2-yl | 2 | H | $C_2H_5$ | $CH_2-CH=CH_2$ | |
| 76 | 1,1-Dioxotetrahydrothien-2-yl | 2 | H | $C_2H_5$ | $C_2H_5$ | |
| 77 | 4,5-Dihydro-4-methylthien-2-yl | 0 | H | $C_3H_7$ | $C_2H_5$ | |
| 78 | 4,5-Dihydro-4-methylthien-2-yl | 0 | H | $C_3H_7$ | $CH_2-CH=CH_2$ | |
| 79 | 4,5-Dihydro-4-methylthien-2-yl | 0 | H | $C_2H_5$ | $CH_2-CH=CH_2$ | |
| 80 | 4,5-Dihydro-4-methylthien-2-yl | 0 | H | $C_2H_5$ | $C_2H_5$ | |
| 81 | 4,5-Dihydro-4,5-dimethylthien-2-yl | 0 | H | $n-C_3H_7$ | $C_2H_5$ | |
| 82 | 4,5-Dihydro-4,5-dimethylthien-2-yl | 0 | H | $n-C_3H_7$ | $CH_2-CH=CH_2$ | |

(Fortsetzung)

| Nr. | $R^4$ | n | $R^1$ | $R^2$ | $R^3$ | $^1H$-NMR (ppm) |
|---|---|---|---|---|---|---|
| 83 | ·,5-Dihydro-4,5-dimeth,lthien-2-yl | 0 | H | $C_2H_5$ | $CH_2-CH=CH_2$ | |
| 84 | 4,5-Dihydro-4,5-dimethylthien-2-yl | 0 | H | $C_2H_5$ | $C_2H_5$ | |
| 85 | 4,5-Dimethyltetrahydrothien-2-yl | 0 | H | $n-C_3H_7$ | $C_2H_5$ | |
| 86 | 4,5-Dimethyltetrahydrothien-2-yl | 0 | H | $n-C_3H_7$ | $CH_2-CH=CH_2$ | |
| 87 | 4,5-Dimethyltetrahydrothien-2-yl | 0 | H | $C_2H_5$ | $CH_2-CH=CH_2$ | |
| 88 | 4,5-Dimethyltetrahydrothien-2-yl | 0 | H | $C_2H_5$ | $C_2H_5$ | |
| 89 | 4,5-Dihydro-4-ethyl-5-propylthien-2-yl | 0 | H | $n-C_3H_7$ | $C_2H_5$ | |
| 90 | 4,5-Dihydro-4-ethyl-5-propylthien-2-yl | 0 | H | $n-C_3H_7$ | $CH_2-CH=CH_2$ | |
| 91 | 4,5-Dihydro-4-ethyl-5-propylthien-2-yl | 0 | H | $C_2H_5$ | $CH_2-CH=CH_2$ | |
| 92 | 4,5-Dihydro-4-ethyl-5-propylthien-2-yl | 0 | H | $C_2H_5$ | $C_2H_5$ | |
| 93 | 4-Ethyl-5-propyltetrahydrothien-2-yl | 0 | H | $n-C_3H_7$ | $C_2H_5$ | |
| 94 | 4-Ethyl-5-propyltetrahydrothien-2-yl | 0 | H | $n-C_3H_7$ | $CH_2-CH=CH_2$ | |
| 95 | 4-Ethyl-5-propyltetrahydrothien-2-yl | 0 | H | $C_2H_5$ | $CH_2-CH=CH_2$ | |
| 96 | 4-Ethyl-5-propyltetrahydrothien-2-yl | 0 | H | $C_2H_5$ | $C_2H_5$ | |
| 97 | 2,5-Dihydrothien-3-yl | 0 | $COOCH_3$ | $n-C_3H_7$ | $C_2H_5$ | 1,00(t,3H); 1,32(t,3H); 4,11(q,2H); 5,68(t,1H) |
| 98 | 2-Methyl-2,5-dihydrothien-3-yl | 0 | H | $n-C_3H_7$ | $C_2H5$ | 0,99(t,3H); 1,34(t,3H); 4,09(q,2H); 5,51(t,1H) |
| 99 | 2,2-Dimethyl-2,5-dihydrothien-3-yl | 0 | H | $n-C_3H_7$ | $CH_2-CH=CHCl$ (trans) | 0,98(t,3H); 1,61(s,6H); 4,51(d,2H); 5,60(t,1H) |
| 100 | 1,1-Dioxo-tetrahydrothien-2-yl | 1 | H | $n-C_3H_7$ | $C_2H_5$ | 0,98(t,3H); 1,17(t,3H); 3,09(m,3H); 4,05(q,2H) |

Die Cyclohexenonderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, z. B. Methanol, Ethanol, Propanol, Butanol, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkohol ykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl- -pyrrolidon und erhält eine Lösung, die zur Anwendung in Form kleinster Tropfen geeignet ist.

II. 20 Gewichtsteile der Verbindung Nr. 53 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid and 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 41 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man ein wäßrige Dispersion, die 0,02 Gewichtsprozent des Wirkstoffs enthält.

V. 20 Gewichtsteile des Wirkstoffs Nr. 19 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer

Hammermühle vermahlen. Durch feines Verteilen der Mischung in 20 000 Gewichtsteilen Wasser erhält man eine Spritzbrühe, die 0,1 Gewichtsprozent des Wirsktoffs enthält.

VI. 3 Gewichtsteile des Wirkstoffs Nr. 53 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 1 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 1 werden mit 41 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 4 kg/ha, vorzugsweise 0,1 bis 3 kg/ha.

Die Wirkung der Cyclohexenonderivate der Formel I auf das Wachstum von Pflanzen aus der Gräserfamilie (Gramineen) und breitblättrigen Kulturpflanzen läßt sich durch Gewächshausversuche zeigen :

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,06 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 0 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Avena fatua (Flughafer), Avena sativa (Hafer), Alopecurus myosuroides (Ackerfuchsschwanz), Echinochloa crus-galli (Hühnerhirse), Lolium multiflorum (Ital. Raygras), Setaria italica (Kolbenhirse), Sinapis alba (Weißer Senf), Triticum aestivum (Weizen).

Als Vergleichsmittel werden die aus EP-OS 0071707 bekannten Verbindungen 2-(1-Ethoxyamino-butyliden)-5-(tetrahydrofuran-3-yl)-cyclohexan-1,3-dion (A) und 2-(1-Ethoxyamino-butyliden)-5-(tetrahydrofuran-2-yl)-cyclohexan-1,3-dion (B) getestet.

Die Aufwandmengen an Vergleichsverbindung entsprechen denjenigen der erfindungsgemäßen Verbindungen.

Bei Vorauflaufanwendung erweisen sich beispielsweise die Verbindungen Nr. 1, 19, 41 bei Aufwandmengen von 3,0 g Wirkstoff/ha als herbizid wirksam gegen Pflanzen aus der Familie der Gräser (Gramineen). Dabei bleibt Sinapis alba als breitblättrige Testpflanze unbeschadet.

Beispielhaft ausgewählte Gräser, wie Avena fatua, Alopecurus myosuroides und Setaria italica, lassen sich im Nachauflaufverfahren mit 0,06 kg Wirkstoff/ha der Verbindungen Nr. 41 und 53 gut bekämpfen. Der Vorteil dieser Verbindungen gegenüber den bekannten Cyclohexenonderivaten liegt darin, daß trotz dieser im Nachauflauf erzielten herbiziden Wirkung gegen Gramineen die Kulturpflanze Weizen aus der gleichen Gräserfamilie nicht geschädigt wird. Im Gegensatz zu den Vergleichsmitteln A und B sind die erfindungsgemäßen Verbindungen somit in dieser Kultur selektiv.

In Anbetracht der Verträglichkeit der möglichen Applikationsmethoden können die erfindungsgemäßen Verbindungen noch in einer weiteren Zahl von Kulturpflanzen zur Beseitigung unerwünschter

Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. naprobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotania tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |

| Botanischer Name | Deutscher Name |
|---|---|
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (post directed) |

Zur Verbreiterung des Wirkungsspektrums und zur Erzielung synergistischer Effekte können die Cyclohexenonderivate der Formel I und ihre Salze mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate anderer Struktur und andere herbizide Wirkstoffe in Betracht.

Außerdem kann es von Nutzen sein, die Cyclohexenon-Derivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behehung von Ernährungs- und Spurenelementmängeln eingesetzt werden. Es können zur Wirkungssteigerung auch nichtphytotoxische Öle und Ölkonzentrate zugesetzt werden.

**Patentansprüche**

1. Cyclohexenonderivate der Formel

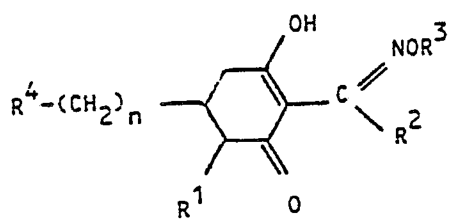

(I)

in der

$R^1$ Wasserstoff oder Alkoxycarbonyl mit 2 bis 5 Kohlenstoffatomen,

$R^2$ Alkyl mit 1 bis 4 Kohlenstoffatomen,

$R^3$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen,

$R^4$ einen nichtaromatischen, gegebenenfalls eine Doppelbindung enthaltenden 5-Ring, der ein Schwefelatom, eine Sulfinyl- oder eine Sulfonylgruppe als Ringglied enthält und gegebenenfalls durch 1 bis 3 Alkylgruppen mit 1 bis 4 Kohlenstoffatomen substituiert ist, und

n 0, 1 oder 2 bedeuten,

mit der Maßgabe, daß n nicht 0 ist, wenn $R^4$ einen 5-Ring mit einer Sulfinyl- oder einer Sulfonylgruppe bedeutet, deren Gleichgewichtsisomere (Tautomere) und Salze dieser Verbindungen.

2. Cyclohexenonderivate der Formel I gemäß Anspruch 1, dadurch gekennzeichnet, daß $R^1$ Wasserstoff bedeutet.

3. Cyclohexenonderivate der Formel I gemäß Anspruch 1, dadurch gekennzeichnet, daß $R^4$ einen nichtaromatischen, ein Schwefelatom und gegebenenfalls eine Doppelbindung enthaltenden 5-Ring bedeutet.

4. Verfahren zur Herstellung eines Cyclohexenonderivates der Formel I gemäß Anspruch 1, dadurch gekennzeichnet, daß man eine Verbindung der Formel

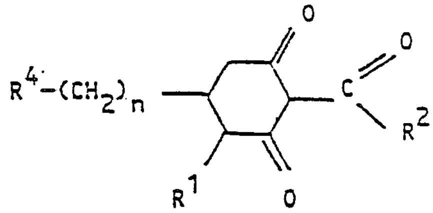

(II)

in der $R^1$, $R^2$, $R^4$ und n die im Anspruch 1 genannten Bedeutungen haben,

a) mit einer Ammoniumverbindung der Formel $R^3O—NH_3Y$, in der $R^3$ die im Anspruch 1 genannten Bedeutungen hat und Y ein Anion bedeutet, in einem inerten Verdünnungsmittel gegebenenfalls in Gegenwart von Wasser bei einer Temperatur zwischen 0 und 80 °C in Gegenwart einer Base oder

b) mit einem gegebenenfalls in wäßriger Lösung vorliegenden Hydroxylamin der Formel $R^3O—NH_2$, in der $R^3$ die in Anspruch 1 genannten Bedeutungen hat, in einem inerten Lösungsmittel umsetzt.

5. Herbizid, enthaltend ein Cyclohexenonderivat der Formel I gemäß Anspruch 1 oder Salze dieser Verbindung.

6. Herbizid, enthaltend inerte Zusatzstoffe und ein Cyclohexenonderivat der Formel I gemäß Anspruch 1 oder Salze dieser Verbindung.

7. Herbizid, enthaltend inerte Zusatzstoffe und ein Cyclohexenonderivat der Formel I gemäß Anspruch 2 oder Salze dieser Verbindung.

8. Herbizid, enthaltend inerte Zusatzstoffe und ein Cyclohexenonderivat der Formel I gemäß Anspruch 3 oder Salze dieser Verbindung.

9. Verfahren zur Bekämpfung unerwünschten Pflanzenwachstums, dadurch gekennzeichnet, daß man die unerwünschten Pflanzen und/oder die von unerwünschtem Pflanzenwachstum freizuhaltende Fläche mit einer herbizid wirksamen Menge eines Cyclohexan-1,3-dionderivates der Formel I gemäß Anspruch 1 oder Salzen dieser Verbindung behandelt.

**Claims**

1. A cyclohexenone derivative of the formula

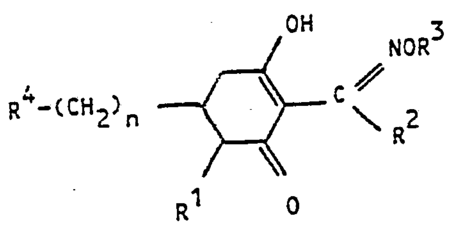

(I)

where

$R^1$ is hydrogen or alkoxycarbonyl of 2 to 5 carbon atoms,

$R^2$ is alkyl of 1 to 4 carbon atoms

$R^3$ is alkyl of 1 to 4 carbon atoms, alkenyl of 3 or 4 carbon atoms, alkynyl of 3 or 4 carbon atoms or haloalkenyl of 3 or 4 carbon atoms and 1 to 3 halogen atoms,

$R^4$ is a non-aromatic 5-membered ring which may or may not contain a double bond, contains a sulfur atom or a sulfinyl or sulfonyl group as a ring member and is unsubstituted or substituted by 1, 2 or 3 alkyl groups of 1 to 4 carbon atoms, and

n is 0, 1 or 2,

with the proviso that n is not 0 when $R^4$ is a 5-membered ring containing a sulfinyl or sulfonyl group, and equilibrium isomers (tautomers) and salts thereof.

2. A cyclohexenone derivative of the formula I as claimed in claim 1, where $R^1$ is hydrogen.

3. A cyclohexenone derivative of the formula I as claimed in claim 1, where $R^4$ is a non-aromatic 5-membered ring which may or may not contain a double bond, and contains a sulfur atom.

4. A process for the preparation of a cyclohexenone derivative of the formula I as claimed in claim 1, wherein a compound of the formula

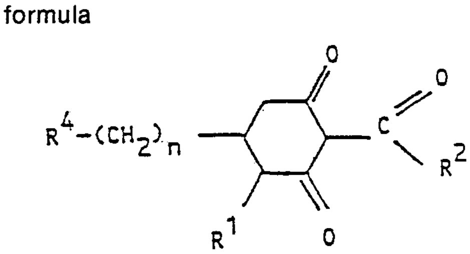

(II)

where $R^1$, $R^2$, $R^4$ and n have the meanings given in claim 1, is reacted with

a) an ammonium compound of the formula $R^3O{-}NH_3Y$, where $R^3$ has the meanings given in claim 1 and Y is an anion, in an inert diluent, in the presence or absence of water, at from 0° to 80 °C, and in the presence of a base, or

b) a hydroxylamine — if desired, in aqueous solution — of the formula $R^3O{-}NH_2$, where $R^3$ has the meanings given in claim 1, in an inert solvent.

5. A herbicide containing a cyclohexenone derivative of the formula I as claimed in claim 1, or a salt thereof.

6. A herbicide containing inert additives and a cyclohexenone derivative of the formula I as claimed in claim 1, or a salt thereof.

7. A herbicide containing inert additives and a cyclohexenone derivative of the formula I as claimed in claim 2, or a salt thereof.

8. A herbicide containing inert additives and a cyclohexenone derivative of the formula I as claimed in claim 3, or a salt thereof.

9. A process for combatting the growth of unwanted plants, wherein the unwanted plants and/or the area to be kept free from unwanted plant growth are treated with a herbicidally effective amount of a cyclohexane-1,3-dione derivative of the formula I as claimed in claim 1, or a salt thereof.

**Revendications**

1. Dérivés de cyclohexenone de formule

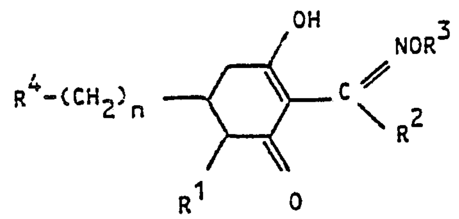

(I)

dans laquelle

$R^1$ représente hydrogène ou alcoxycarbonyle à 2 à 5 atomes de carbone,

$R^2$ représente alkyle à 1 à 4 atomes de carbone,

$R^3$ alkyle à 1 à 4 atomes de carbone, alcényle à 3 à 4 atomes de carbone, alcinyle à 3 à 4 atomes de carbone ou halogénoalcényle à 3 à 4 atomes de carbone et 1 à 3 atomes d'halogène

$R^4$ un noyau pentagonal, non aromatique, contenant éventuellement une double liaison, qui contient, comme terme, un atome de soufre, un groupe sulfinyle ou un groupe sulfonyle et qui est substitué éventuellement par 1 à 3 groupes alkyle à 1 à 4 atomes de carbone, et

n représente 0, 1 ou 2,

sous réserve que n ne soit pas 0 quand $R^4$ représente un noyau pentagonal à un groupe sulfinyle ou un groupe sulfonyle, leurs isomères d'équilibre (tantomères) et les sels de ces composés.

2. Dérivés de cyclohexenone de formule I, selon la revendication 1, caractérisés par le fait que $R^1$ représente hydrogène.

3. Dérivés de cyclohexenone de formule I, selon la revendication 1, caractérisés par le fait que $R^4$ représente un noyau pentagonal, non aromatique, contenant un atome de soufre et éventuellement une double liaison.

4. Procédé de préparation d'un dérivé de cyclohexenone de formule I, selon la revendication 1, caractérisé par le fait que l'on fait réagir, dans un solvant inerte, un composé de formule

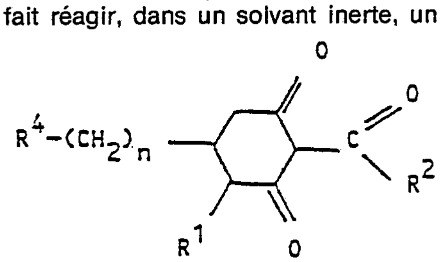

(II)

dans laquelle $R^1$, $R^2$, $R^4$ et n ont les significations données dans la revendication 1,

a) avec un composé d'ammonium de formule $R^3O{-}NH_3Y$, dans laquelle $R^3$ a la signification donnée dans la revendication 1 et Y représente un anion, dans un diluent inerte, éventuellement en présence d'eau, à une température comprise entre 0 et 80 °C, en présence d'une base, ou

b) avec une hydroxylamine de formule $R^3O{-}NH_2$, dans laquelle $R^3$ a la signification donnée dans la revendication 1, se trouvant éventuellement en solution aqueuse.

5. Herbicide contenant un dérivé de cyclohexenone de formule I, selon la revendication 1 ou des sels de ce composé.

6. Herbicide contenant des additifs inertes et un dérivé de cyclohexenone de formule I, selon la revendication 1 ou des sels de ce composé.

7. Herbicide contenant des additifs inertes et un dérivé de cyclohexenone de formule I, selon la revendication 2 ou des sels de ce composé.

8. Herbicide contenant des additifs inertes et un dérivé de cyclohexenone de formule I, selon la revendication 3 ou des sels de ce composé.

9. Procédé pour lutter contre la croissance des plantes indésirables, caractérisé par le fait que l'on traite les plantes indésirables et/ou les surfaces à maintenir exemptes de croissance de plantes indésirables, avec une quantité active, du point de vue herbicide, d'un dérivé de cyclohexane-1,3-dione de formule I selon la revendication 1 ou des sels de ce composé.